# FASCICULE DE BREVET EUROPEEN

(11) **EP 1 003 488 B1**
(45) Date de publication et mention de la délivrance du brevet: **05.01.2005**
(21) Numéro de dépôt: 98940304.3
(22) Date de dépôt: 21.07.1998
(51) Int. Cl.: A61K 9/51

(54) **PROCEDE DE PREPARATION DE NANOCAPSULES DE TYPE VESICULAIRE**
VERFAHREN ZUR HERSTELLUNG VON NANOKAPSELN DES VESIKULÄREN TYPS
METHOD FOR PREPARING VESICULAR NANOCAPSULES

(30) Priorité: 24.07.1997 FR 9709672
(43) Date de publication de la demande: 31.05.2000
(73) Titulaire: UNIVERSITE CLAUDE BERNARD, F-69622 Villeurbanne Cédex (FR)
(72) Inventeur: QUINTANAR, David, Mexico (MX); FESSI, Hatem, F-69003 Lyon (FR); DOELKER, Eric, CH-1231 Conches (CH); ALLEMANN, Eric, CH-1257 Croix-de-Rozon (CH)
(74) Mandataire: Sarlin, Laure V.
(86) Numéro de dépôt international: PCT/FR1998/001611
(87) Numéro de publication internationale: WO 1999/004766

(56) Documents cités:
- EP-A- 0 451 082
- WO-A-96/35414
- DE-A- 19 545 257
- FR-A- 2 084 199

## Description

### DOMAINE TECHNIQUE DE L'INVENTION :

Le domaine technique de l'invention est celui des nanoparticules destinées, en particulier, à l'encapsulation de principes actifs de nature médicamenteuse, cosmétique, diététique, phytosanitaire ou autre. Il s'agit donc, en d'autres termes, de systèmes colloïdaux transporteurs notamment de principes actifs. De tels sytèmes colloïdaux préservent ces principes actifs et peuvent permettre leur libération contrôlée et/ou prolongée au niveau de leur site d'action. Ces systèmes peuvent également avoir comme vocation de masquer le goût et/ou de diminuer la toxicité de certains principes actifs.

Plus précisément, la présente invention concerne la préparation de nanocapsules susceptibles de comprendre au moins un principe actif, ces nanocapsules étant des vésicules dont l'enveloppe est constituée par au moins un matériau macromoléculaire.

La présente invention vise également les nanocapsules telles qu'obtenues par ce procédé, les suspensions colloïdales comprenant ces nanocapsules, ainsi que les compositions, par exemple thérapeutiques, comprenant lesdites nanocapsules en suspension colloïdale ou non.

### ETAT DE LA TECHNIQUE :

On distingue trois familles de vecteurs submicroscopiques permettant le transport de principes actifs, à savoir :
les liposomes, les nanosphères et les nanocapsules.

Les liposomes sont des vésicules submicroniques dont la paroi est constituée d'un ou plusieurs feuillets, comprenant chacun un bicouche à base de phospholipides. Les liposomes présentent notamment les inconvénients suivants :
a) Instabilité physique
b) Instabilité chimique des phospholipides constitutifs (par ex. hydrolyses ou formation de peroxides)
c) Mauvaise reproductibilité de lot à lot
d) Perte du principe actif

Les nanosphères correspondent à des structures de type matriciel formées par des sphères solides, dans lesquelles le principe actif est piégé et/ou dissous.

Les nanocapsules sont des vésicules comportant une enveloppe généralement de nature macromoléculaire. Le ou les principe(s) actif(s) sont susceptibles d'être contenus dans le noyau délimité par ladite enveloppe et/ou adsorbés sur l'enveloppe des vésicules.

La littérature scientifique relative à la caractérisation et à la préparation de ces vecteurs nanoparticulaires colloïdaux de transport de principes actifs, est riche.

A titre d'illustration de la famille des nanosphères, on peut évoquer le brevet américain N° 5 118 528, qui décrit des nanoparticules matricielles à base de polymères et obtenues selon une technique, dans laquelle :
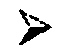 d'une part, on dissout un matériau filmogène constitué par un polymère d'acide lactique dans de l'acétone (phase I)
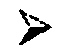 et, d'autre part, on prépare un plus grand volume d'une deuxième phase liquide (phase II) consistant essentiellement en un non-solvant du matériau filmogène, ce non-solvant étant, par exemple, de l'eau.

Les tensioactifs peuvent être ajoutés à la phase 1 contenant le polymère et/ou à la phase II aqueuse. Ils sont par exemple constitués par un polypropylène-glycol et par un tensioactif non ionique ajouté dans la phase II aqueuse. Selon ce procédé, les phases I et II sont mises en présence sous agitation magnétique à 100 tours/min. Le mélange formé est opalescent, ce qui témoigne de la formation de nanoparticules ou vésicules sphériques de polymères d'acide lactique de diamètre environ égal à 200 nanomètres. L'acétone est éliminé de cette suspension colloïdale de nanoparticules par tirage sous vide. On peut également éliminer l'eau par ultrafiltration et séchage, pour produire une poudre de nanosphères. En complément ou en remplacement de l'eau dans la phase II, on peut utiliser un alcool tel que l'éthanol. Ces nanosphères matricielles ne donnent pas entièrement satisfaction, notamment au regard des points suivants :
a) faible rendement
b) nécessité de grands volumes de solvant
c) difficulté pour contrôler la taille de particules.

Il est également possible de préparer des nanosphères matricielles, en mettant en oeuvre une technique d'extraction du solvant du polymère constitutif desdites nanosphères. Cette technique est fondée sur la restauration de la miscibilité du solvant du polymère, dans le solvant formant la phase continue homogène de la suspension colloïdale. Il s'agit de la technique de relargage « salting out ». Elle est décrite notamment dans le brevet US N° 4 968 350. Le procédé décrit dans ce brevet consiste à préparer une première solution 1 aqueuse concentrée d'un solide du type électrolyte (par exemple MgCl₂) à laquelle est ajoutée une quantité suffisante d'un colloïde protecteur (par exemple alcool polyvinylique), de façon à produire une solution visqueuse ou un gel. On réalise par ailleurs une seconde solution II à base d'un polymère non hydrosoluble et formant le matériau constitutif des nanosphères. Ce polymère est par exemple l'acétate de cellulose ou l'acétophtalate de cellulose. Le solvant mis en oeuvre est l'acétone. On mélange ensuite les deux solutions I et II l'une à l'autre sous agitation, de manière à obtenir une émulsion de la solution II de polymère dans le gel aqueux d'alcool polyvinylique + MgCl₂. Les nanosphères sont obtenues grâce à l'extraction de l'acétone de la phase I hétérogène polymère vers la phase II continue aqueuse. Ce transfert est obtenu par addition d'eau dans l'émulsion, ce qui provoque la restauration de conditions physicochimiques permettant que l'acétone et l'eau soient miscibles. La concentration en MgCl₂ est réduite en deçà d'une concentration limite. Selon une variante, on ne prévoit pas d'agent du type MgCl₂ insolubilisant l'acétone dans la solution II de polymère. Dans ce cas, cette dernière est directement émulsifiée dans l'hydrocolloïde. Les sels et solvants organiques peuvent être éliminés par filtration à flux tangentiel afin de récupérer les nanocapsules sous forme d'une dispersion colloïdale aqueuse ou sous forme de poudre par centrifugation/séchage de la dispersion.

Les nanosphères obtenues par « salting out » souffrent naturellement des inconvénients attachés à ce type de nanoparticules vecteurs de principes actifs. Il s'agit entre autres :
a) usage de grandes quantités d'acétone et de sels
b) longues étapes de purification
c) incompatibilité possible entre le sel et certains composés bioactifs
d) grandes quantités d'alcool polyvinylique résiduel, ce qui n'est pas acceptable pour une administration intraveineuse.

Les nanocapsules ont ceci d'avantageux sur les nanosphères et les liposomes, qu'elles permettent une meilleure protection des principes actifs incorporés ou encapsulés, de même qu'un meilleur contrôle de leur libération in vivo. En outre, elles permettent l'incorporation de grandes quantités de principes actifs huileux.

Parmi les techniques traditionnelles de préparation de nanocapsules, la polymérisation interfaciale figure en bonne place. Cette technique est une polymérisation de monomères à l'interface d'une émulsion eau dans huile ou huile dans eau. Le polymère se développe en formant une paroi autour des globules de phase hétérogène, ce qui conduit finalement à une enveloppe encapsulant ladite phase hétérogène contenant ou non le principe actif. On produit ainsi une multitude de vésicules en suspension dans la phase continue.
Classiquement les polymères d'enveloppe des nanocapsules sont des polymères acryliques, méthacryliques ou alkyles cyano(meth)acryliques. Le brevet US N° 4 329 332 divulgue la préparation de nanocapsules par introduction du monomère alkylcyanoacrylate, dans une solution aqueuse d'un agent tensioactif de préférence non ionique, tel que le monolaurate de polyhydroxyéthyl-sorbitan. Le milieu est ensuite soumis à une agitation vigoureuse, de manière à former une solution micellaire. Le pH de cette solution est ajusté à 2-3, à l'aide d'un acide physiologiquement acceptable. L'adjonction de l'éventuel principe actif se fait par dissolution dans la phase aqueuse réactionnelle. Les nanocapsules obtenues ont une taille d'environ 200 nanomètres. En outre, il est à noter que le pH acide de la phase aqueuse peut être dommageable à un certain nombre de principes actifs. Les nanocapsules selon ce brevet ont également pour inconvénient de faire intervenir des monomères, qui sont potentiellement toxiques à l'état de résidus. Ces nanocapsules ont également ceci de désavantageux, qu'une réaction chimique est possible entre le polymère et certains principes actifs.

La demande de brevet PCT WO 94/15 590 décrit des nanocapsules dont le polymère d'enveloppe est constitué par un alkyl-2-cyanopolyacrylate (alkyl = 4-ter-octyl-phényl-, 2'-carboxyéthyl-, hexadécyl-,). Selon ce procédé, on utilise des monomères alkylcyanoacrylates hydrophiles (ester d'acide cyanoacrylique avec du polyéthylène glycol) ou lipophiles (hexadécyl-2-cyanoacrylate) et on met en oeuvre des systèmes mono ou biphasiques. Dans les systèmes monophasiques, le principe actif constitue lui-même la phase dispersée servant de support à la polymérisation qui conduit aux nanocapsules. Dans les systèmes biphasiques faisant intervenir deux liquides non miscibles l'un à l'autre, le principe actif est contenu dans l'une des phases, l'ensemble formant la phase dispersée de l'émulsion, siège de la polymérisation interfaciale.

La demande PCT WO 94 17789 divulgue plus précisément des nanocapsules obtenues par polymérisation interfaciale, dans un système biphasique.

Le brevet US N° 5 500 224 divulgue une composition pharmaceutique comprenant des suspensions colloïdales de nanocapsules préparées par polymérisation interfaciale de n-butyle-2-cyanoacrylate sur des micelles comportant du tampon acide acétique pH 4,3 et un tensioactif du type lanrylsulfate de sodium. La phase huileuse continue est constituée par du mygliol (triglycéride d'acide gras en C₈-C₁₀) additionné de monooléate de sorbitanne (SPAN 80).
Les nanocapsules obtenues ont un diamètre de l'ordre de 250 nanomètres. Le principe actif est contenu dans la phase aqueuse dispersée formant le coeur des nanocapsules.

La polymérisation interfaciale permet également de préparer des nanosphères.

Les nanoparticules obtenues par polymérisation interfaciale présentent un certain nombre d'inconvénients, dont certains sont évoqués ci-après.
Dans certains cas, cette technique fait intervenir des solvants organiques qui sont pharmaceutiquement rédhibitoires en raison de leur haute toxicité. Il est alors indispensable de purifier les nanocapsules en éliminant ces solvants indésirables. Cela complique beaucoup le procédé et grève lourdement son coût de revient.

En outre, cette technique peut nécessiter l'emploi de macromolécules du type sérum albumine ou dextrane, qui présentent le grave défaut d'être immunogène.

De plus, les alkylcyanoacrylates sont des polymères d'enveloppe dont la biocomptabilité laisse encore à désirer. De surcroît, la toxicité des monomères utilisés pour préparer ces polymères, est certaine, ainsi que celle des produits obtenus après biodégradation.

Par ailleurs, la technique de polymérisation interfaciale est relativement contraignante donc coûteuse notamment au regard des conditions de polymérisation.

Pour être complet, on signalera également l'existence du brevet FR 2 084 199, qui concerne l'obtention non pas de nano mais de microcapsules, par la technique de « salting out ». Il s'agit donc, selon ce brevet, de préparation de microgranulés sphériques s'écoulant librement, solides, et comprenant une matrice polymère enrobant des particules liquides ou solides de substances encapsulées. Ce procédé comprend les étapes suivantes :
a - préparation d'une solution du polymère d'encapsulage (éthylcellulose) dans un solvant (n-butanol) soluble à 15 % en poids **au plus** dans l'eau à 20° C ;
b - dissolution ou dispersion dans la solution a, d'un matériau à enrober solide ou liquide, par exemple de l'huile de paraffine ;
c - dispersion de la solution ou de l'émulsion obtenue (phase organique A) dans un liquide aqueux (phase aqueuse B = eau saturée en n-butanol) non miscible avec le solvant organique ;
d - migration lente et sous contrôle du solvant organique (n-butanol) de la phase organique A dans la phase aqueuse B, de façon que le polymère dissous soit déposé à la surface des particules dispersées dans B, cette migration s'opérant par incorporation d'eau dans le milieu ;
e - séparation et éventuellement séchage des particules solidifiées, c'est-à-dire le mélange de granulés.
Les vésicules obtenues sont des microcapsules de diamètre compris entre 80 et 500 µ. Ce sont des vésicules constituées par un noyau à base d'huile de paraffine enveloppée d'éthyle cellulose. Il est clair que le solvant n-butanol est mis en oeuvre dans ce procédé sous forme pure et non sous forme d'une solution d'eau dans le n-butanol. Par ailleurs, ce procédé est contraignant en ce qu'il impose de choisir un solvant dont la solubilité dans l'eau n'excède pas 15 % en poids à 20 °C.

### BREF EXPOSE DE L'INVENTION :

Dans cet état de la technique, l'un des objectifs essentiels de la présente invention est de fournir un procédé de préparation de nanocapsules chargées ou non en principes actifs et formées par des vésicules dont l'enveloppe est constituée par au moins un polymère, lesdites nanocapsules se devant :
- d'être dispersibles en phase liquide, par exemple aqueuse, sous forme colloïdale,
- d'être exemptes de produits toxiques,
- d'être constituées par des substances biocompatibles et éventuellement biodégradables,
- d'être stables en suspension colloïdale,
- de permettre une bonne protection du principe actif éventuellement encapsulé, de même qu'une libération prolongée et/ou contrôlée de celui-ci in vivo,
- et d'être obtenable de manière simple et économique.

Un autre objectif essentiel de la présente invention est de fournir un procédé de préparation de nanocapsules du type de celui sus-visé et qui soit en outre aisé et rentable à mettre en oeuvre à l'échelle industrielle.

Un autre objectif de l'invention est de fournir un procédé de préparation de nanocapsules du type visé supra permettant d'obtenir de manière fiable et reproductible des particules de taille inférieure à 1 µm.

Un autre objectif essentiel de la présente invention est de fournir un procédé de préparation de nanocapsules du type visé supra et ne nécessitant pas d'étapes de purification lourdes et coûteuses.

Un autre objectif essentiel de la présente invention est de fournir un procédé de préparation de nanocapsules de type visé supra et permettant l'encapsulation d'une grande variété de principes actifs de nature hydrophile ou lipophile.

S'étant fixée tous ces objectifs, la demanderesse a eu le mérite de mettre en évidence, de manière tout à fait surprenante et inattendue, qu'il est possible de préparer des nanocapsules
- en faisant intervenir deux phases non huileuses, non miscibles l'une à l'autre et une troisième phase huileuse ;
- en réalisant une émulsion huile dans eau, dont la phase dispersée huileuse contient le polymère d'enveloppe ;
- et en extrayant le solvant du polymère d'enveloppe contenu dans la phase dispersée lipophile en faisant en sorte que les conditions de la phase continue soient telles que le solvant du polymère d'enveloppe redevienne au moins en partie miscible dans la phase continue.

Grâce à ces dispositions, la demanderesse a pu observer avec étonnement, après de nombreux travaux et essais, que la solidification du polymère se produisait à l'interface de l'émulsion et se développait en formant les parois des nanocapsules renfermant la phase hétérogène.

Il s'ensuit que les objectifs fixés au départ, parmi d'autres, ont pu être atteints par la présente invention, qui concerne un procédé de préparation de nanocapsules **(NC)** susceptibles de comprendre au moins un principe actif **(PA),** ces nanocapsules étant des vésicules de taille moyenne inférieure à 1000 nm et dont l'enveloppe est constituée par au moins un polymère **(PE),**
caractérisé en ce qu'il consiste essentiellement :
- 1 -à mettre en oeuvre au moins trois substances liquides **(S**_{**1**}**, S**_{**2**}**, S**_{**3**}**)**, au moins un polymère d'enveloppe **(PE)**, et éventuellement un ou plusieurs principes actifs **(PA),**
   ces substances étant choisies de telle sorte que :
   * **S**_{**1**} est un solvant/dispersant organique, au moins partiellement miscible avec **S**_{**2**} et **S**_{**3**}, et agissant comme solvant et/ou dispersant de **PE** et de l'éventuel **PA,**
   * **S**_{**2**} est un non-solvant de **PE,** non miscible à **S**_{**3**},
   * **S**_{**3**} est un liquide, de préférence une phase huileuse, non-solvant de **PE** et solvant/dispersant de l'éventuel **PA** ; **S**_{**3**} étant en outre destiné à rentrer dans la constitution du coeur des **NC** ;
- 2 - à réaliser une première phase liquide homogène I comprenant une solution de **PE,** de **S**_{**3**} et, éventuellement de **PA** dans **S**_{**1**} **; S**_{**1**} étant apporté par l'intermédiaire d'une solution de **S**_{**2**} dans **S**_{**1**}, de préférence saturée en **S**_{**2**}.
- 3 - à préparer une deuxième phase liquide homogène II, comprenant le solvant **S**_{**2**} et dans laquelle **S**_{**1**} peut être émulsifiée,
- 4 - à mettre en présence les phases 1 et II, en leur adjoignant éventuellement au moins un tensioactif stabilisant,
- 5 - à procéder à la mise en émulsion du mélange obtenu à l'étape 4, pour produire une émulsion de I dans II,
- 6 - à ajouter du solvant **S**_{**2**} dans l'émulsion I/II, de façon à instaurer dans la phase homogène II, des conditions telles que **S**_{**1**} soit au moins en partie miscible à **S**_{**2**} et permettre ainsi la diffusion de **S**_{**1**} de la phase 1 dans la phase II, pour obtenir in fine les NC en suspension dans une nouvelle phase continue III,
- 7 - éventuellement à éliminer tout ou partie de **S**_{**1**} et/ou **S**_{**2**},

Le procédé selon l'invention s'articule autour du choix du solvant/dispersant **S**_{**1**} et du non solvant **S**_{**2**}, ainsi que sur la miscibilité partielle de **S**_{**1**} et de **S**_{**2**}.
Un autre fondement de l'invention tient à la réalisation d'une émulsion
■ dont la phase dispersée contient le polymère d'enveloppe, le solvant/dispersant **S**_{**1**}, l'éventuel PA et l'huile **S**_{**3**}
■ et dont la phase continue contient principalement **S**_{**2**}, les conditions étant telles que **S**_{**1**} n'est plus soluble dans **S**_{**2**}.

Le procédé de l'invention est également basé sur la disposition sélun laquelle on met fin à la non miscibilité de **S**_{**1**} dans **S**_{**2**} après la réalisation de l'émulsion, de façon à tirer **S**_{**1**} de la phase dispersée vers la phase continue et permettre ainsi la solidification du polymère d'enveloppe et subséquemment la formation des nanocapsules. Il ne reste plus alors qu'à éliminer **S**_{**1**} et tout ou partie de **S**_{**2**}.

Enfin, la caractéristique préférée de saturation mutuelle des solvants **S**_{**1**}**, S**_{**2**} dans l'étape ②, favorise, contre toute attente, l'obtention des nanoparticules de taille moyenne inférieure au micron.

### EXPOSE DETAILLE DE L'INVENTION

Un tel procédé est commode à mettre en oeuvre. Il conduit à des nanocapsules de taille moyenne inférieure à 1000 nanomètres, de préférence à 500 nanomètres. La stabilité en suspension colloïdale de ces nanocapsules est avérée. Elle confère au principe actif qu'elles sont susceptibles d'encapsuler soit dans leur coeur, soit par adsorption sur leur paroi, une protection au stockage ainsi que lors de leur transport vers leur site d'action. Ces nanoparticules sont en effet tout à fait appropriées pour être utilisées comme système colloïdal de vectorisation de principes actifs, notamment pharmaceutiques. Ces nanocapsules ne sont pas toxiques aussi bien au stade du produit fini qu'au stade méthodologique au travers des matériaux et dispositifs mis en oeuvre pour leur obtention.
Le fait d'utiliser une huile, une émulsion du type huile dans eau et une solidification de la coque polymère par une extraction de solvant reposant sur l'instauration de conditions de solubilisation ou de miscibilité dans la phase continue non huileuse, sont des éléments tout à fait novateurs qui traduisent le mérite et le caractère inventif du procédé ici considéré.
L'activité inventive est encore renforcée par le fait qu'il n'était absolument pas prédictible que le polymère se solidifie à l'interface selon une structure pariétale. La solidification aurait pu, par exemple, intervenir dans la masse de la phase hétérogène, de préférence huileuse.

Les notions de solubilité et de miscibilité auxquelles on se réfère, à titre d'exemple non limitatif, dans le cadre de la présente sont explicitées notamment au chapitre 14 page 340 et suivantes de l'ouvrage PHARMACOPEE GMP.

On reviendra plus en détail ci-après sur l'étape ① du procédé selon l'invention consistant dans le choix des matières premières **S**_{**1**}**, S**_{**2**}**, S**_{**3**}**, PE** et éventuellement **PA.**

Dans l'étape ②, on prépare une première phase liquide homogène I en dissolvant le polymère d'enveloppe **PE** et le liquide **S**_{**3**}, de préférence huileux, et éventuellement le principe actif **PA** dans le solvant **S**_{**1**}.
Conformément à l'invention, ce dernier peut être employé pur ou être constitué par une solution du non-solvant **S**_{**2**} dans **S**_{**1**}.
Dans un tel cas de figure, il est préférable (sans que cela ne soit limitatif) que cette solution de **S**_{**2**} dans **S**_{**1**}, soit saturée en **S**_{**2**}. Cela rend plus commode l'étape ③ d'émulsification de la phase I dans la phase II, puisque cela supprime le temps de latence induit par l'amenée à saturation de **S**_{**2**} dans **S**_{**1**}. La saturation mutuelle des solvants favorise le processus d'émulsification.

En pratique, cette étape ②, est réalisée de manière conventionnelle, dans des conteneurs, de préférence équipés de moyens d'agitation. Cette étape s'effectue avantageusement à température ambiante et à pression atmosphérique.

L'étape ③ du procédé selon l'invention est celle au cours de laquelle, on prépare la deuxième phase liquide homogène II. Cette phase II est caractérisée en ce qu'elle comprend le solvant **S**_{**2**} et en ce qu' elle constitue un milieu dans lequel **S**_{**1**} et plus généralement la phase I peut être émulsifiée.
Selon une variante 3' de cette étape ③, on prépare une phase II comprenant exclusivement **S**_{**2**} ou constituée par une solution de **S**_{**1**} dans **S**_{**2**}**, S**_{**1**} n'étant pas à saturation. Dans un tel cas de figure, on fait en sorte que dans l'étape ⑤, les quantités de phase I et II (Volumes totaux en **S**_{**1**} et **S**_{**2**}) mises en oeuvre soient en proportions telles que se forme l'émulsion de 1 dans II. Dans cette variante 3', il faut donc tout d'abord que la phase I mélangée à la phase II, amène **S**_{**1**} à saturation dans **S**_{**2**}, avant que l'émulsion I/II se produise. Cela allonge d'autant l'étape ③ comme dans le cas évoqué ci-dessus, où la phase I n'est pas formée par une solution non-saturée de **S**_{**2**} dans S₁.
Dans une variante 3" de l'étape ③, on prépare une phase II à base de **S**_{**2**} et comprenant :
- a - soit **S**_{**1**} à saturation
- b - soit au moins un agent **A** apte à rendre **S**_{**1**} non miscible à **S**_{**2**}
- c - soit la combinaison de a et de b.
Dans cette variante, les sous-variantes a, b, c ont pour vocation d'améliorer la formation de l'émulsion et, dans le meilleur des cas, de permettre à celle-ci de se produire instantanément après le mélange des phases I et II.
En tout état de cause conformément à l'invention, on privilégie le mode opératoire selon lequel, lors du mélange des phases I et II, le solvant **S**_{**1**} et le non-solvant **S**_{**2**} ne sont que partiellement miscibles l'un à l'autre.
En pratique, on utilise, de préférence, dans la phase 1 une solution de **S**_{**1**} saturée en **S**_{**2**} et dans la phase II une solution de **S**_{**2**} saturée en **S**_{**1**}.
L'étape ③ se déroule idéalement avec les mêmes moyens matériels et dans les mêmes conditions de température et de pression que l'étape ②. Il en va d'ailleurs de même en ce qui concerne les étapes ④ et ⑤ de mise en présence et d'émulsification des phases 1 et II.

Selon une disposition avantageuse de l'invention, la mise en émulsion ⑤ est réalisée sous agitation vigoureuse, de préférence à l'aide de moyens mécaniques fonctionnant à un régime ≥ 1500 tr/min, de préférence ≥ 5 000 tr/min et, plus préférentiellement encore compris entre 7 000 et 10 000 tr/min.
A titre d'exemple de dispositif d'émulsification convenable pour le procédé selon l'invention, on peut citer un agitateur mécanique à hélice ou un homogénéisateur (ultra-Turrax®)

La formation des nanocapsules **NC** par solidification du polymère **PE** à l'interface de l'émulsion I dans II, se produit lors de l'étape ⑥. Il s'agit dans cette étape, de faire en sorte que **S**_{**1**} devienne miscible à **S**_{**2**}.
De manière préférée, on parvient à cela en complétant l'émulsion I/II à l'aide du solvant **S**_{**2**}.
Selon une variante envisageable dans le cas où l'on met en oeuvre dans la phase II, un agent A apte à rendre **S**_{**1**} non miscible à **S**_{**2**}, il est possible de prévoir la formation de NC par dilution de la phase externe au moyen d'eau et d'éliminer A par un moyen approprié (par ex. filtration à flux tangentiel).

Une fois réalisé le transfert de **S**_{**1**} dans la phase homogène II, on procède dans l'étape ⑦, à l'élimination de tout ou partie de **S**_{**1**} et/ou **S**_{**2**}. Avantageusement on élimine **S**_{**1**} par tout moyen approprié tel que le soutirage par le vide plus ou moins poussé, l'évaporation, la distillation ou tout autre moyen de fractionnement.
Après élimination de **S**_{**1**} de la phase II, on obtient une suspension colloïdale de nanocapsules **NC** dans **S**_{**2**}. Cette suspension peut être plus ou moins concentrée par élimination et/ou ajout de **S**_{**2**}.
Les moyens mis en oeuvre pour éliminer **S**_{**2**} peuvent être les mêmes que ceux employés pour retirer **S**_{**1**}_{.} En pratique, on peut par exemple purifier la suspension par filtration à flux tangentiel, de manière à recueillir les nanocapsules **NC** que l'on peut ensuite sécher, de manière à les produire sous forme sèche et solide. Il est également tout-à-fait envisageable de procéder à une atomisation ou à une lyophilisation (en présence ou non d'un agent cryoprotecteur) des nanocapsules **NC** en suspension.

Ces nanocapsules **NC** sont parfaitement caractérisées et possèdent une structure stable et bien définie. Une fois formées, elles confèrent à la suspension un aspect blanc laiteux à reflets bleutés tout à fait révélateur et spécifique.
Ces nanocapsules possèdent également des densités et des vitesses de sédimentation spécifiques, qui permettent de les distinguer des autres nanoparticules, telles que les nanosphères comprises dans les nanoémulsions.

La stabilité de la suspension colloïdale selon l'invention est d'au moins une année.
Avantageusement, la température de mise en oeuvre du procédé est comprise entre 4 et 45° C de préférence entre 15 et 25° C.

Selon une disposition préférée de l'invention, on choisit les proportions en **S**_{**1**}**, S**_{**2**}**, S**_{**3**} et **PE,** de telle sorte que la taille des **NC** soit inférieure à 1000nm, ces proportions étant, de préférence, les suivantes (exprimées en % par rapport à **S**_{**1**}) :
* **PE** compris entre 0,1 et 100, de préférence entre 0,5 et 20 poids sec/volume,
* **S**_{**2**} compris entre 51 et 1000, de préférence entre 100 et 500 V/V,
* **S**_{**3**} compris entre 0,1 et 10, de préférence entre 0,1 et 5,0 V/V.
En tout état de cause, on fait en sorte que, sur le plan quantitatif, la proportion de **S**_{**2**} soit la plus faible possible de manière à disposer d'une suspension concentrée de nanocapsules. Il en va de même en ce qui concerne les quantités de **S**_{**1**} mises en oeuvre, de manière à faciliter l'élimination de ce dernier.

Les nanocapsules **NC** produites par le procédé selon l'invention ont avantageusement une taille contrôlée inférieure à 1000 nanomètres, de préférence à 500 nanomètres, et plus préférentiellement comprise entre 50 et 350 nanomètres.

S'agissant des produits mis en oeuvre dans le procédé conforme à l'invention, on précise ci-après, sans que cela ne soit limitatif, les groupes de substances **S**_{**1**}**, S**_{**2**}**, PE** et **PA,** vers lesquels le choix se porte de préférence.

Le solvant **S**_{**1**} est avantageusement sélectionné dans la famille des alcools, phénols, cétones, esters d'acides carboxyliques et leurs mélanges. Plus précisément, on retient particulièrement le groupe de produits suivants pour **S**_{**1**} :
acétate d'éthyle, alcool benzylique, propylène carbonate, butanol,
butanone et leurs mélanges,

Le non-solvant **S**_{**2**} est avantageusement un liquide ou un mélange de liquides contenant de l'eau et/ou au moins un alcool. Ainsi **S**_{**2**} est sélectionné plus spécialement dans le groupe de produits suivants :
eau, alcool - par exemple éthanol, propylène-glycol ou glycérine - et leurs mélanges.

Le liquide **S**_{**3**}, de préférence, huileux est choisi dans la famille des huiles végétales ou minérales, huiles neutres, huiles essentielles, acides gras, esters d'acides carboxyliques, terpènes, vitamines et leurs mélanges.
Avantageusement, on retiendra les produits huileux suivants :
Mygliol®. 810, 812 et 840 (Dynamit Nobel, Germany), Labrafac® lipophilique, Lauroglycol (Gattefossé, France), huile minérale, huile d'olive, huile de sésame, huile de maïs, huile de coton, huile de cacahuète, benzoate de benzyle, myristate d'isopropyle, huile essentielle de lavande, huile essentielle de bouquet, vitamine E, clofibrate, etc.

En ce qui concerne les polymères d'enveloppe **PE**, la sélection s'opère parmi les homo et/ou copolymères naturels et synthétiques biocompatibles. Il peut s'agir par exemple de l'acide polylactique D ou L et DL, copolymères d'acide lactique et d'acide glycolique ; la poly ε-caprolactone ; la polypropiolactone ; la polybutylrolactone ; la polypivalactone ; l'acétate butyrate de cellulose ; l'éthylcellulose ; la phtalate d'hydroxyméthylpropytcellulose ; la gomme laque ; l'acéto-phtale de polyvinyle ; l'acétophtalate de cellulose ; les acrylates et les polymères acryliques (Eudragit®, Röhm Pharma, Germany) ; les polymères obtenus à partir d'esters cycliques des acides hydroxybutyrique, hydroxyisobutyrique, hydroxyméthylvalérique, phényl-lactique, hydroxyéthylbutyrique, le polybétamalate de benzyle, les polycyanocrylates d'alkyle, les polyéthylène-vinyl acétate ; et leurs mélanges.
Conformément à l'invention **PE** est choisi dans le groupe de polymères suivants :
- (co)polymère d'acide(s)α-hydrocarboxylique(s), de préférence l'acide lactique et/ou l'acide glycolique,
- (co)polymère d'acide (meth)acrylique et/ou de méthacrylate,
- poly-ε-caprolactone,
- cellulose et ses dérivés
- bloc polymère d'acide α-hydroxycarboxylique et du poly(oxyde d'éthylène)
- cyanocrylates,
- et copolymères et/ou mélanges d'entre eux.

Les principes actifs **PA** susceptibles d'être encapsulés dans les nanocapsules **NC** préparées conformément à l'invention, peuvent être divers et variés. La condition à respecter quant au choix du principe actif tient à sa solubilité et/ou à son aptitude à la dispersion dans le solvant **S**_{**1**}**.** De manière générale, il peut s'agir par exemple de principes actifs utilisés en allergologie, anesthésiologie, cancerologie, cardiologie et angiologie, dermatologie, endocrinologie, gastroentérologie, gynécologie, hematologie, hepatologie, immunologie, infectrologie, neurologie, ophtalmologie, parasitologie, pneumologie, rhumatologie, stomatologie, toxicologie, ou utilisés en tant qu'antalgiques ou anti-inflammatoires...

De préférence, le principe actif est choisi dans le groupe de produits suivants :
indométacine, hormones, - de préférence progestérone-Estradiol-, Chlorambucil, **S**_{**3**}, vitamines (de préférence vit E et K), cyclosporine A, ibuprofen, propanolol, acide valproïque, clofibrate, etc, et leurs mélanges.

Concernant les tensioactifs stabilisants convenables pour l'étape ④ du procédé selon l'invention, on les choisit de préférence conformément à l'invention parmi les tensioactifs ioniques ou non ioniques. Plus précisément, le choix peut se porter par exemple sur les alcools polyvinyliques par exemple Mowiol® 4-88 (Hoechst, Frankfurt, Germany) ; les poloxamers par exemple Pluronics® F-68 et F-127 (BASF, Wyandotte, EU) ; les sels biliaires par exemple glycocolate de sodium et les sels d'acides carboxyliques par exemple oléate de sodium.

Les avantages du procédé selon l'invention, sont nombreux. On peut citer entre autres :
- fiabilité,
- haut rendement,
- reproductibilité,
- transposition d'échelle facile pour des applications industrielles.
- mise en oeuvre d'équipements conventionnels non sophistiqués (en particulier les homogénéisateurs à haute pression ou les dispositifs d'ultrasonication ne sont pas indispensables)
- utilisation de solvants, de stabilisants et d'additifs non toxiques et bien tolérés par l'organisme.
- contrôle de la taille des nanocapsules **NC** obtenues.

Selon un autre de ces aspects, la présente invention vise également les nanocapsules **NC** *per se* telles qu'elles sont obtenues par le procédé ci-dessus défini ou par tout autre procédé conduisant au même résultat ou à un résultat semblable. En particulier, l'invention a pour objet des nanocapsules **NC** de taille intérieure à 1000 nanomètres, de préférence à 500 nanomètres, dont la paroi est constituée par au moins un polymère **PE** tel que présenté supra et qui comprenne, à l'état de trace ou non, des substances **S**_{**1**} et/ou **S**_{**2**} et/ou **S**_{**3**} et éventuellement un principe actif **PA** ; ces substances étant telles que définies supra.

La présente invention vise également une suspension colloïdale de nanocapsules **NC** caractérisée en ce qu'elle est obtenue à partir du produit issu de l'étape ⑥ du procédé tel que défini ci-dessus et/ou en redispersant les nanocapsules **NC** obtenus à l'issue de l'étape ⑦ dudit procédé, dans un non-solvant de **PE.**

Un autre objet de l'invention est constitué par une composition thérapeutique comprenant les **NC** chargés en **PA** et obtenus par le procédé tel que présenté ci-dessus ou par un procédé conduisant au même produit.

La présente invention sera mieux comprise à la lumière des exemples donnés ci-après. Ces derniers feront également ressortir tous les avantages et les variantes de mise en oeuvre du procédé selon l'invention. En outre, ces exemples comprendront la caractérisation et l'évaluation des propriétés des nanocapsules NC conformes à la présente invention.

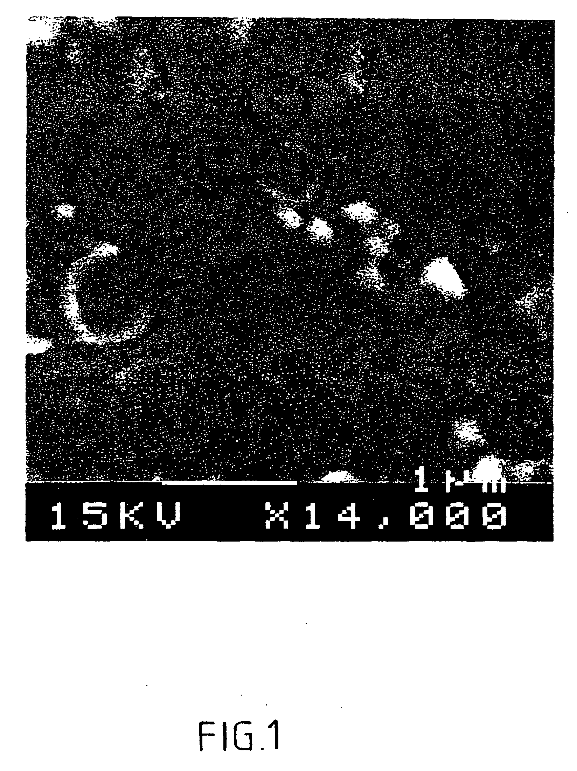

Pour compléter l'illustration donnée par les exemples, on fournit en annexe, une **Figure 1** unique représentant une photographie au microscope électronique à balayage des nanocapsules selon l'invention - grossissement : x 14.000.

### EXEMPLES

### EXEMPLE 1 : PREPARATION DE NANOCAPSULES DE POLYMERE BIODEGRADABLE CONTENANT UN LIQUIDE ORGANIQUE

D'une part, 200 mg de polymère **PE** d'acide lactique D,L (MEDISOPB® 100 DL) et 0,5 ml de triglycérides d'acides caprilyque/caprique - huile S₃ - (Mygliol® 812) sont dissous dans 20 ml d'acétate d'éthyle **S**_{**1**} saturé avec de l'eau = **S**_{**2**} (Phase 1).
D'autre part, 2 g d'alcool polyvinylique (Mowiol® 4-88), agent tensioactif, sont dissous dans 40 ml d'eau (**S**_{**2**}) purifiée saturée avec de l'acétate d'éthyle **S**_{**1**} (Phase II).
La phase I à base de **S**_{**1**} saturé avec **S**_{**2**} (acétate d'éthyle + eau), est émulsionnée dans la phase aqueuse II (**S**_{**2**}) sous agitation vigoureuse (environ 8000tr/min) pendant dix minutes. 200 ml d'eau = **S**_{**2**} sont ajoutés à l'émulsion sous agitation à fin de permettre la diffusion de l'acétate d'éthyle vers la phase II aqueuse. Le mélange (phase III) devient blanc laiteux avec des reflets bleutés dûs à la formation des nanocapsules **NC** dont la paroi est constitué de **PE.**
L'acétate d'éthyle **S**_{**1**} est éliminé sous pression réduite (vide de la trompe à eau) et la suspension est concentrée, par élimination de l'eau **S**_{**2**} dans les mêmes conditions, jusqu'au volume désiré.
La taille des nanocapsules **NC** mesurée dans un diffractomètre à rayon laser (Nanosizer® de la firme Coultronics) est de 329 nm avec un indice de dispersion de 2. L'existence des nanocapsules **NC** a été confirmée, d'une part, par cryofracture des nanocapsules et observation par microscopie électronique à balayage (Fig. 1) et, d'autre part, par la comparaison de la vitesse de sédimentation sous ultracentrifugation des nanocapsules selon l'invention et de nanoémulsions ou de nanosphères témoins préparées par la même méthode à la différence près que pour les nanoémulsions, il n'y a pas de polymère **PE** dans la préparation et pour les nanosphères, il n'y a pas d'huile **S**_{**3**} dans la préparation. En effet, la vitesse de sédimentation de nanoémulsions, nanosphères et nanocapsules diffère en raison des constitutions différentes et par conséquent de leur densité. On peut mesurer la densité de ces systèmes par centrifugation isopycnique (sur un gradient de densité de silice colloïdale (Pereoll®, Pharmacia, LKB, Suède). La centrifugation a été faite à 4° C et 1500 g pendant 3 heures. Des tubes marqueurs de densités connues (Sephadex®, Pharmacia) ont été utilisés pour calculer la densité des systèmes. Après centrifugation, elle est mesurée par la distance (h) entre le ménisque de la dispersion et la bande contenant les nanoparticules.

### Résultats :

Nanoémulsions : Il n'y a pas sédimentation (h = 0)
Considérant que la densité du Mygliol® 812 est de 0,9438 g/cm³ (calculée avec un pycnomètre) il est logique que l'huile contenue dans la nanoémulsion tende à flotter.

Nanosphères : Sédimentation (h = 55,12 mm).
Densité calculée = 1,1405 g/cm³
La forte sédimentation démontre la constitution matricielle solide des particules.

Nanocapsules : Sédimentation (h = 14,98 mm)
Densité calculée = 1,0357 g/cm³
Cette sédimentation indique un état intermédiaire entre une nanoémulsion et des nanosphères.
Ceci indique que le polymère et l'huile font partie des particules.
Le fait qu'il y ait seulement une bande confirme la structure vésiculaire dans laquelle l'huile forme le noyau.

### EXEMPLE 2 : PREPARATION DE NANOCAPSULES CONTENANT DES HUILES COSMETIQUES

On procède comme indiqué dans l'exemple 1 mais en substituant le Mygliol® 812 par une huile minérale dans la phase I d'acétate d'éthyle/eau.
Les nanocapsules **NC** ont une taille de 303 nm avec un indice de dispersion de 2.

### EXEMPLE 3 : PREPARATION DE NANOCAPSULES CONTENANT UNE ESSENCE

On procède comme indiqué dans l'exemple I, mais en substituant le Mygliol® 812 par 0, 1 ml d'huile essentielle de lavande au niveau de la phase I d'acétate d'éthyle.
Les nanocapsules **NC** ont une taille de 304 nm avec un indice de dispersion de 2.

### EXEMPLE 4 : PREPARATION DE NANOCAPSULES CONTENANT UN COLORANT LIPOPHILE

On procède comme indiqué dans l'exemple I, mais en ajoutant 5 mg de Soudan III dans la phase I d'acétate d'éthyle.
Les nanocapsules ont une taille de 340 nm avec un indice de dispersion de 2.
La suspension des nanocapsules est ensuite centrifugée à 20 000 tr/min pendant 40 min. Le dépôt est séché sous vide en dessiccateur. Environ 30 mg du produit sec sont dissous dans 50 m de chloroforme. L'absorbance de la solution est mesurée à 518 nm en référence à une courbe de calibration. Le pourcentage de Soudan III encapsulé (rapporté au pourcentage du contenu initial) est de 100,8 %.

### EXEMPLE 5 : PREPARATION DE NANOCAPSULES AVEC UN POLYMERE DE SOLUBILITE DEPENDANT DU pH

On procède comme indiqué dans l'exemple 1, mais en substituant le polymère PE et le solvant **S**_{**1**} respectivement par de l'Eudragit® E (polymère acrylique soluble à pH gastrique) et par du propylène carbonate.
Les nanocapsules **NC** ont une taille de 239 nm avec un indice de dispersion de 3.

### EXEMPLE 6 : PREPARATION DE NANOCAPSULES AVEC UN POLYMERE DE SOLUBILITE DEPENDANT DU pH, CONTENANT UN COLORANT LIPOPHILE

On procède comme indiqué dans l'exemple 4, mais en substituant le polymère **PE** et le solvant **S**_{**1**} par de l'Eudragit® E et par de l'alcool benzylique, respectivement.
Les nanocapsules ont une taille de 287 nm avec un indice de dispersion de 2.
Le pourcentage de Soudan III encapsulé après filtration tangentielle (dispositif Minitan®) est de 92,4 %.

### EXEMPLE 7 : PREPARATION DE NANOCAPSULES CONTENANT UN PRINCIPE ACTIF SOLIDE

On procède comme indiqué dans l'exemple I, mais en ajoutant 20 mg d'indométacine dans la phase I d'acétate d'éthyle. **S**_{**1**} sature en eau **S**_{**2**}.
Les nanocapsules ont une taille de 314 nm avec un indice de dispersion de 2.
La suspension est centrifugée et séchée comme dans l'exemple 4. Environ 30 mg du produit sec sont dissous dans 20 ml de chloroforme. Après une dilution convenable avec du chloroforme, l'absorbance est, mesurée à 248 nm en référence à une courbe de calibration. Le pourcentage d'indométacine encapsulée est de 94,4 %.

### EXEMPLE 8 : PREPARATION DE NANOCAPSULES CONTENANT UN PRINCIPE ACTIF LIQUIDE

On procède comme indiqué dans l'exemple 1, mais en remplaçant l'huile **S**_{**3**} (mygliol) par du clofibrate **S**_{**3**}. Les nanocapsules ont une taille de 317 nm avec un indice de dispersion de 2.
La suspension est centrigée et sechée comme dans l'exemple 4.
Environ 25 mg du matériel sec sont dissous dans 20 ml de chloroforme. L'absorbance est ensuite mesurée à 280 nm en référence à une courbe de calibration. Le pourcentage de clofibrate encapsulé est de 95,3 %.

### EXEMPLE 9 : PREPARATION DE NANOCAPSULES CONTENANT UN ACTIF COSMETIQUE

On procède comme indiqué dans l'exemple 1, mais en substituant l'huile à de la vitamine E pour former **S**_{**3**}. Les nanoparticules ont une taille de 322 nm, avec un indice de dispersion de 2.
La suspension est centrifugée et séchée comme dans l'exemple 4.
Environ 20 mg du produit sec sont dissous dans 20 ml de chloroforme. L'absorbance est ensuite mesurée à 297 nm, en référence à une courbe de calibration. Le pourcentage de vitamine E encapsulée est de 92,2 %.

## Revendications

1. Procédé de préparation de nanocapsules **(NC)** susceptibles de comprendre au moins un principe actif **(PA),** ces nanocapsules étant des vésicules de taille moyenne inférieure à 1000 nm et dont l'enveloppe est constituée par au moins un polymère **(PE),**
**caractérisé en ce qu'**il consiste essentiellement :
- 1 - à mettre en oeuvre au moins trois substances liquides **(S**_{**1**}**, S**_{**2**}**, S**_{**3**}**)**, au moins un polymère d'enveloppe **(PE),** et éventuellement un ou plusieurs principes actifs **(PA),** ces substances étant choisies de telle sorte que :
* **S**_{**1**} est un solvant/dispersant organique, au moins partiellement miscible avec **S**_{**2**} et **S**_{**3**}, et agissant comme solvant et/ou dispersant de **PE** et de l'éventuel **PA,**
* **S**_{**2**} est un non-solvant de **PE,** non miscible à **S**_{**3**},
* **S**_{**3**} est un liquide, de préférence une phase huileuse, non-solvant de PE et solvant/dispersant de l'éventuel **PA** ; **S**_{**3**} étant, en outre, destiné à rentrer dans la constitution du coeur des **NC** ;
- 2 - à réaliser une première phase liquide homogène I comprenant une solution de **PE,** de **S**_{**3**} et, éventuellement de **PA** dans **S**_{**1**} ; **S**_{**1**} étant apporté par l'intermédiaire d'une solution de **S**_{**2**} dans **S**_{**1**}, de préférence saturée en **S**_{**2**}.
- 3 - à préparer une deuxième phase liquide homogène II, comprenant le solvant **S**_{**2**} et dans laquelle **S**_{**1**} peut être émulsifiée,
- 4 - à mettre en présence les phases I et II, en leur adjoignant éventuellement au moins un tensioactif stabilisant,
- 5 - à procéder à la mise en émulsion du mélange obtenu à l'étape 4, pour produire une émulsion de I dans II,
- 6 - à ajouter du solvant **S**_{**2**} dans l'émulsion I/II de façon à instaurer dans la phase homogène II, des conditions telles que **S**_{**1**} soit au moins en partie miscible à **S**_{**2**} et permettre ainsi la diffusion de **S**_{**1**} de la phase I dans la phase II, pour obtenir in fine les **NC** en suspension dans une nouvelle phase continue III,
- 7 - éventuellement à éliminer tout ou partie de **S**_{**1**} et/ou **S**_{**2**}.

2. Procédé selon la revendication 1, **caractérisé en ce que**, dans l'étape ③, on prépare une phase II comprenant exclusivement **S**_{**2**} ou constituée par une solution de **S**_{**1**} dans **S**_{**2**}**, S**_{**1**} n'étant pas à saturation et **en ce que**, dans l'étape ⑤, on met en oeuvre des quantités de phases I et II (Volumes totaux en **S**_{**1**} et **S**_{**2**}) dans des proportions choisies de telle sorte que se forme une émulsion de I dans II.

3. Procédé selon la revendication 1, **caractérisé en ce que**, dans l'étape ③, on prépare une phase II à base de **S**_{**2**} et comprenant :
- a - soit **S**_{**1**} à saturation
- b - soit au moins un agent **A** apte à rendre **S**_{**1**} non miscible à **S**_{**2**}
- c - soit la combinaison de a et de b.

4. Procédé selon l'une quelconque des revendications 1 à 3, **caractérisé en ce que** l'étape ⑤ de mise en émulsion est réalisée par agitation mécanique à un régime ≥ 1500tr/min.

5. Procédé au moins selon l'une quelconque des revendications 1 à 4, **caractérisé en ce que** l'étape ⑦ (facultative) d'élimination de tout ou partie de **S**_{**1**} et/ou de **S**_{**2**} est réalisée par évaporation et/ou filtration tangentielle, et/ou (ultra)centrifugation et/ou atomisation, et/ou soutirage par le vide.

6. Procédé selon l'une quelconque des revendications 1 à 5, **caractérisé en ce que** l'on choisit les proportions en **S**_{**1**}**, S**_{**2**}**, S**_{**3**} et **PE,** de telle sorte que la taille des NC soit inférieure à 1000nm, ces proportions étant, de préférence, les suivantes (exprimées en % par rapport à **S**_{**1**}) :
* **PE** compris entre 0,1 et 100, de préférence entre 0,5 et 20 poids sec/volume,
* **S**_{**2**} compris entre 51 et 1000, de préférence entre 100 et 500 V/V,
* **S**_{**3**} compris entre 0,1 et 10, de préférence entre 0,1 et 5,0 V/V.

7. Procédé selon l'une quelconque des revendications 1 à 6, **caractérisé en ce que** l'on sélectionne :
→ **S**_{**1**} dans le groupe de produits suivants :
acétate d'éthyle, alcool benzylique, propylène carbonate, butanol, butanone et leurs mélanges,
→ **S**_{**2**} dans le groupe de produits suivants :
eau, alcool - par exemple éthanol, propylène-glycol ou glycérine - et leurs mélanges,
→ **S**_{**3**} dans le groupe de produits huileux suivants :
Mygliol® 810, 812 et 840 (Dynamit Nobel, Germany), Labrafac® lipophilique, Lauroglycol (Gattefossé, France), huile minérale, huile d'olive, huile de sésame, huile de mais, huile de coton, huile de cacahuète, benzoate de benzyle, myristate d'isopropyle, huile essentielle de lavande, huile essentielle de bouquet, vitamine E, clofibrate,
→ **PE** dans le groupe de polymères suivants :
• (co)polymères d'acide(s)α-hydrocarboxylique(s), de préférence l'acide lactique et/ou l'acide glycolique,
• (co)polymère d'acide (meth)acrylique et/ou de méthacrylate,
• poly-ε-caprolactone,
• cellulose et ses dérivés
• bloc polymère d'acide α-hydroxycarboxylique et du poly(oxyde d'éthylène)
• cyanocrylates,
• et copolymères et/ou mélanges d'entre eux.
→ **PA** dans le groupe de produits suivants :
indométacine, hormones, - de préférence progestérone-Estradiol-, Chlorambucil, **S**_{**3**}, vitamines (de préférence vit E et K), cyclosporine A, ibuprofen, propanolol, acide valproïque, clofibrate et leurs mélanges.

## Claims

1. Procedure for the preparation of nanocapsules (NC) likely to comprise at least an active constituent **(AC),** these nanocapsules being vesicles of an average size of less than 1000 nm and whose envelope consists of at least one polymer **(PE)**,
**characterised in that** it consists mainly :
- 1-of using at least three liquid substances **(S**_{**1**}**, S**_{**2**}**, S**_{**3**}**)**, at least one envelope polymer **(PE),** and possibly one or more active constituents **(AC),** these substances being selected in such a way that :
* **S**_{**1**} is an organic solvent/dispersant, at least partly miscible with **S**_{**2**} and **S**_{**3**}, and acting as a solvent and/or dispersant of **PE** and of the possible **AC,**
* **S**_{**2**} is a non-solvent of PE, not miscible with **S**_{**3**},
* **S**_{**3**} is a liquid, preferably an oily phase, non-solvent of PE and solvent/dispersant of the possible **AC** ; **S**_{**3**} being also intended to enter the constitution of the core of the **NCs** ;
- 2-of producing a first homogeneous liquid phase I comprising a solution of **PE,** of **S**_{**3**}, and possibly of **AC** in **S**_{**1**}; **S**_{**1**} being brought through a solution of **S**_{**2**} in **S**_{**1**}, preferably saturated with **S**_{**2**},
- 3-of preparing a second homogeneous liquid phase II, comprising the solvent **S**_{**2**} and in which **S**_{**1**} can be emulsified,
- 4-of placing in presence phases I and II, possibly adding to them at least one stabilising tensioactive substance,
- 5-of carrying out the emulsioning of the mixture obtained in stage 4, to produce an emulsion of I in II,
- 6-of adding some solvent **S**_{**2**} to the emulsion I/II, so as to form in the homogeneous phase II, conditions such that **S**_{**1**} is at least partly miscible with **S**_{**2**}, and thus to permit the diffusion of **S**_{**1**} of phase I in phase II, to obtain finally the **NCs** in suspension in a new continuous phase III,
- 7-possibly to eliminate all or part of **S**_{**1**} and/or of **S**_{**2**}.

2. Procedure according to claim 1, **characterised in that**, in stage ③, a phase II is prepared comprising solely **S**_{**2**} or consisting of a solution of **S**_{**1**} in **S**_{**2**}**, S**_{**1**} not being in saturation and **in that**, in stage ⑤, use is made of the quantities of phases I and II (Total volumes of **S**_{**1**} and **S**_{**2**}) in proportions selected so that an emulsion of I is formed in II.

3. Procedure according to claim 1, **characterised in that**, in stage ⑤, a phase II based on **S**_{**2**} is prepared and comprising:
- a-either **S**_{**1**} at saturation point
- b-either at least an agent A able to render **S**_{**1**} not miscible with **S**_{**2**}
- c-or a combination of a and b.

4. Procedure according to any of claims 1 to 3, **characterised in that** stage ⑤ of emulsioning is carried out by mechanical agitation at a speed ≥ 1500 rpm.

5. Procedure at least according to any one of claims 1 to 4, **characterised in that** (optional) stage ⑦ of elimination of all or part of **S**_{**1**} and/or of **S**_{**2**} is achieved by evaporation and/or by tangential filtration, and/or (ultra)centrifugation and/or atomisation, and/or drawing under vacuum.

6. Procedure according to any one of claims 1 to 5, **characterised in that** the proportions of **S**_{**1**}**, S**_{**2**}**, S**_{**3**} and **PE** are selected in such a way that the size of the **NCs** is less than 1000 nm, these proportions being, preferably, as follows (expressed as % relative to **S**_{**1**}) :
* **PE** included between 0.1 and 100, preferably between 0.5 and 20 dry weight/volume,
* **S**_{**2**} included between 51 and 1000, preferably between 100 and 500 V/V,
* **S**_{**3**} included between 0.1 and 10, preferably between 0.1 and 5.0 V/V.

7. Procedure according to any one of claims 1 to 6, **characterised in that** the following are selected:
- **S**_{**1**} in the following group of products:
ethyl acetate, benzylic alcohol, carbonate propylene, butanol, butanone and their mixtures,
- **S**_{**2**} in the following group of products:
water, alcohol - e.g. ethanol, propylene-glycol or glycerine - and their mixtures,
- **S**_{**3**} in the following groups of oily products:
Mygliol ®810, 812 and 840 (Dynamit Nobel, Germany), Labrafac ® Lipophilique, Lauroglycol (Gattefosse, France), mineral oil, olive oil, sesame oil, corn oil, cotton oil, groundnut oil, benzyl benzoate, isopropyl myristate, essential oil of lavender, essential oil of bouquet, vitamin E, clofibrate,
- **PE** in the following polymer groups:
. a-hydrocarboxylic acid (co)polymers, preferably lactic acid and/or glycolic acid,
. (co)polymer of (meth)acrylic acid and/or of methacrylate,
. poly-e-caprolactone,
. cellulose and its derivatives,
. polymer block of a-hydroxycarboxylic acid and of ethylene polyoxyde,
. cyanocrylates,
. and copolymers and/or mixtures of them.
- **AC** in the following group of products:
indometacine, hormones - preferably progesterone-Estradiol-,Chlorambucil, **S**_{**3**}, vitamins (preferably vit. E and K), cyclosporine A, ibuprofen, propanolol, valproic acid, clofibrate and their mixtures.

## Patentansprüche

1. Verfahren zur Herstellung von Nanokapseln (NK), die geeignet sind mindestens einen Wirkstoff (WS) zu enthalten, wobei diese Nanokapseln Bläschen von einer mittleren Größe von weniger als 1000 nm sind und deren Hülle aus mindestens einem Polymer (PE) besteht,
**dadurch gekennzeichnet, dass** es im Wesentlichen daraus besteht, dass:
- 1 - mindestens drei flüssige Substanzen (S₁, S₂, S₃), mindestens ein umhüllendes Polymer (PE) und eventuell ein oder mehrere Wirkstoffe (WS) bereitgestellt werden,
wobei diese Substanzen so ausgewählt sind, dass:
* S₁ ein organisches Lösungs-/Dispergierungsmittel ist, das mindestens teilweise mischbar mit S₂ und S₃ ist,
und als Lösungs- und/oder Dispergierungsmittel von PE und dem eventuellen WS dient,
* S₂ eine unlösliche Substanz von PE und nicht mischbar mit S₃ ist,
* S₃ eine Flüssigkeit ist, vorzugsweise eine unlösliche Ölphase von PE und löslich/dispergierend vom eventuellen WS; wobei S₃ außerdem dafür bestimmt ist, sich am Aufbau des Kerns der NK zu beteiligen;
- 2 - eine erste flüssige homogene Phase I ausgeführt wird, bestehend aus einer Lösung von PE, S₃ und eventuell von WS in S₁ ausgeführt wird; wobei S₁ mittels einer Lösung von S₂ in S₁, vorzugsweise gesättigt mit S₂,
- 3 - eine zweite flüssige homogene Phase II zubereitet wird, die mindestens das Lösungsmittel S₂ enthält und in der S₁ emulgiert werden kann,
- 4 - die Phasen I und II zusammengebracht werden, indem ihnen eventuell mindestens ein stabilisierender spannungsaktiver Stoff hinzugefügt wird,
- 5 - das in Schritt 4 erhaltene Gemisch emulgiert wird, um eine Emulsion von I in II herzustellen,
- 6 - Lösungsmittel S₂ zur Emulsion I/II hinzugefügt wird, um in der homogenen Phase II solche Bedingungen zu schaffen, dass S₁ zumindest teilweise mit S₂ mischbar ist und so die Verteilung von S₁ der Phase I in die Phase II zu ermöglichen, um schließlich die NK in Suspension in einer neuen kontinuierlichen Phase III zu erhalten,
- 7 - S₁ und/oder S₂ eventuell ganz oder teilweise eliminiert wird.

2. Verfahren nach Anspruch 1, **dadurch gekennzeichnet, dass** in Schritt 3 eine Phase II zubereitet wird, die ausschließlich S₂ enthält oder aus einer Lösung von S₁ in S₂ besteht, wobei S₁ nicht gesättigt ist, und dadurch, dass in Schritt 5 Mengen der Phasen I und II (Gesamtvolumen von S₁ und S₂) in Verhältnissen ausgeführt werden, die so gewählt sind, dass eine Emulsion von I in II entsteht.

3. Verfahren nach Anspruch 1, **dadurch gekennzeichnet, dass** in Schritt 3. eine Phase II auf der Basis von S₂ zubereitet wird, die Folgendes enthält:
- a- entweder S₁ in Sättigung
- b - oder mindestens eine Substanz A, die bewirken kann, dass S₁ nicht mischbar mit S₂ ist
- c - oder die Kombination von a und b.

4. Verfahren nach einem der Ansprüche 1 bis 3, **dadurch gekennzeichnet, dass** in Schritt 5 die Emulgierung durch mechanisches Bewegen im Bereich von ≥ 1500 U/Min. durchgeführt ist.

5. Verfahren zumindest nach einem der Ansprüche 1 bis 4, **dadurch gekennzeichnet, dass** Schritt 7 (fakultativ), d.h., die ganze oder teilweise Eliminierung von S₁ und/oder S₂ durch Verdampfung und/oder tangentiale Filterung, und/oder (Ultra)zentrifugation und/oder Zerstäubung, und/oder Vakuumabziehen ausgeführt ist.

6. Verfahren nach einem der Ansprüche 1 bis 5, **dadurch gekennzeichnet, dass** die Verhältnisse von S₁, S₂, S₃ und PE so gewählt sind, dass die Größe der NK unter 1000 nm beträgt, wobei diese Verhältnisse vorzugsweise die folgenden sind (ausgedrückt in % in Bezug auf S₁) :
* PE zwischen 0,1 und 100, vorzugsweise zwischen 0,5 und 20 Trockengewicht/Volumen,
* S₂ zwischen 51 und 1000, vorzugsweise zwischen 100 und 500 V/V,
* S₃ zwischen 0,1 und 10, vorzugsweise zwischen 0,1 und 5,0 V/V.

7. Verfahren nach einem der Ansprüche 1 bis 6, **dadurch gekennzeichnet, dass** folgende Auswahl getroffen ist:
- > S₁ aus der Gruppe folgender Produkte:
Ethylacetat, Benzylalkohol, Propylenkarbonat, Butanol, Butanon und deren Gemische.
- > S₂ aus der Gruppe folgender Produkte:
Wasser, Alkohol - z.B. Ethanol, Propylenglykol oder Glyzerin - und deren Gemische.
- > S₃ aus der Gruppe folgender Ölprodukte:
Mygliol® 819, 812 und 840 (Dynamit Nobel, Deutschland), lipophiles Labrafac®, Lauroglycol (Gattefossé, Frankreich), Mineralöl, Olivenöl, Sesamöl, Maisöl, Baumwollöl, Erdnussöl, Benzylbenzoat, Isopropylmyristat, ätherisches Lavendelöl, ätherisches Bouquetöl, Vitamin E, Clofibrat.
- > PE aus der Gruppe folgender Produkte:
* (Co)polymeren von α-Kohlenwasserstoffsäure(n), vorzugsweise Milchsäure und/oder Glykolsäure,
* (Co)polymer von (Meth)acrylsäure und/oder Methacrylat,
* Poly-ε-Caprolacton,
* Zellulose und deren Derivate,
* Blockpolymer von α-Kohlenwasserstoffsäure und Poly(ethylenoxid),
* Cyanocrylaten,
* und Copolymeren und/oder Gemischen davon.
- > WS aus der Gruppe folgender Produkte:
Indometacin, Hormone, - vorzugsweise Progesteron-Estradiol-, Chlorambucil, S₃, Vitamine (vorzugsweise Vitamin E und K), Cyclosporin A, Ibuprofen, Propanolol, Valproinsäure, Clofibrat und deren Gemische.
